# EUROPEAN PATENT APPLICATION

(11) **EP 4 534 531 A1**
(43) Date of publication of application: **09.04.2025**
(21) Application number: 23839456.3
(22) Date of filing: 27.06.2023
(51) Int. Cl.: C07D 249/08, C07B 57/00

(54) **METHOD FOR PRODUCING TRIAZOLE DERIVATIVE ENANTIOMER (R)**

(30) Priority: 14.07.2022 JP 2022113477
(71) Applicant: Kureha Corporation, Chuo-ku Tokyo 103-8552 (JP)
(72) Inventor: HAGIHARA, Ryusuke, Tokyo 103-8552 (JP); UMENO, Tomohiro, Machida-shi, Tokyo 194-8543 (JP); KARASAWA, Satoru, Machida-shi, Tokyo 194-8543 (JP)
(74) Representative: Hoefer & Partner Patentanwälte mbB
(86) International application number: PCT/JP2023/023822
(87) International publication number: WO 2024/014282

(57) **Abstract**

Provided is a method for producing an (R)-enantiomer of a triazole derivative. The method includes adding a chiral molecule represented by General Formula (IIa) or (IIb) to a triazole derivative represented by General Formula (I) in a solvent to perform crystallization, and separating a precipitated crystal and a residual liquid.

## Description

### TECHNICAL FIELD

The present invention relates to a method for producing an (R)-enantiomer of a triazole derivative.

### BACKGROUND ART

There has been a need for agricultural or horticultural chemicals having low toxicity to humans and animals and excellent handling safety, and exhibiting a high controlling effect against a wide variety of plant diseases. Under such circumstances, Patent Document 1 discloses a triazole derivative having high antimicrobial activity against phytopathogenic fungi, and an agricultural or horticultural chemical and a protective agent for an industrial material containing the same as an active ingredient. In addition, Patent Document 2 discloses an (-)-enantiomer of a triazole derivative having higher activity.

### Citation List

### Patent Document

Patent Document 1: WO 2019/093522
Patent Document 2: WO 2021/230382

### SUMMARY OF INVENTION

### TECHNICAL PROBLEM

Patent Document 2 discloses a preparative separation method from the racemate of a triazole derivative using a column of optical resolution as a method for preparing a dominantly active (-)-enantiomer. However, from an industrial point of view, a method for obtaining a specific enantiomer by means other than a column has been demanded to reduce costs.

The present invention has been made in view of the above problems, and an object of the present invention is to provide a method for producing a specific enantiomer of a triazole derivative.

### SOLUTION TO PROBLEM

As a result of intensive studies, the present inventors have found that addition of a specific chiral molecule to the racemate of a triazole derivative can allow co-crystallization of the chiral molecule with a (-)-enantiomer. From a result of other studies, the present inventors have found that the (-)-enantiomer of the triazole derivative is an (R)-enantiomer of the triazole derivative, leading to the present invention.

That is, to solve the above problems, a method for producing an (R)-enantiomer of a triazole derivative according to one aspect of the present invention includes adding a chiral molecule represented by the following General Formula (IIa) or (IIb) to a triazole derivative represented by the following General Formula (I) in a solvent to perform crystallization, and separating a precipitated crystal and a residual liquid: 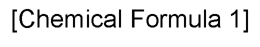 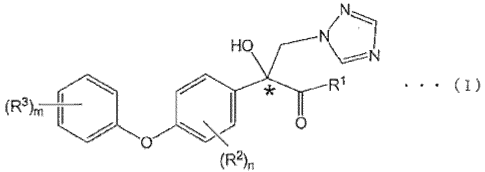 where in Formula (I),
R¹ is -OR⁴ or -NR⁵R⁶;
R⁴, R⁵, and R⁶ are each independently hydrogen, a C₁-C₆-alkyl group, a C₂-C₆-alkenyl group, a C₂-C₆-alkynyl group, a C₃-C₈-cycloalkyl group, a C₃-C₈-cycloalkyl-C₁-C₄-alkyl group, a phenyl group, a phenyl-C₁-C₄-alkyl group, a phenyl-C₂-C₄-alkenyl group, or a phenyl-C₂-C₄-alkynyl group, and R⁵ and R⁶ may form a ring together with a nitrogen atom to which they are bonded; the aliphatic groups in R⁴, R⁵, and R⁶ may have 1, 2, 3 or a possible maximum number of the same or different groups R^{a}s, where R^{a}s are each independently selected from a halogen group, a cyano group, a nitro group, a C₁-C₄-alkoxy group, and a C₁-C₄-haloalkoxy group;
R² is a halogen group, a cyano group, a nitro group, a phenyl group, a phenyl-oxy group, a C₁-C₄-alkyl group, a C₁-C₄-haloalkyl group, a C₁-C₄-alkoxy group, a C₁-C₄-haloalkoxy group, -SOR⁷, or -SFs;
R³ is a halogen group, a cyano group, a nitro group, an amino group, a phenyl group, a phenyl-oxy group, a C₁-C₄-alkyl group, a C₁-C₄-haloalkyl group, a C₁-C₄-alkoxy group, a C₁-C₄-haloalkoxy group, a C₁-C₄-alkylamino group, a C₁-C₄-dialkylamino group, a C₁-C₄-alkylacylamino group, -SOR⁷, or -SFs; the cycloalkyl group and phenyl group moieties in R⁴, R⁵, and R⁶ and the phenyl group moiety in R³ may have 1, 2, 3, 4, 5, or a possible maximum number of the same or different groups R^{b}s, where R^{b}s are each independently selected from a halogen group, a cyano group, a nitro group, a C₁-C₄-alkyl group, a C₁-C₄-alkoxy group, a C₁-C₄-haloalkyl group, and a C₁-C₄-haloalkoxy group;
R⁷ is a C₁-C₄-alkyl group or a C₁-C₄-haloalkyl group;
n is 0, 1, 2, 3, or 4;
m is 1, 2, 3, 4, or 5; and
the asterisk (*) refers to a chiral carbon atom, and 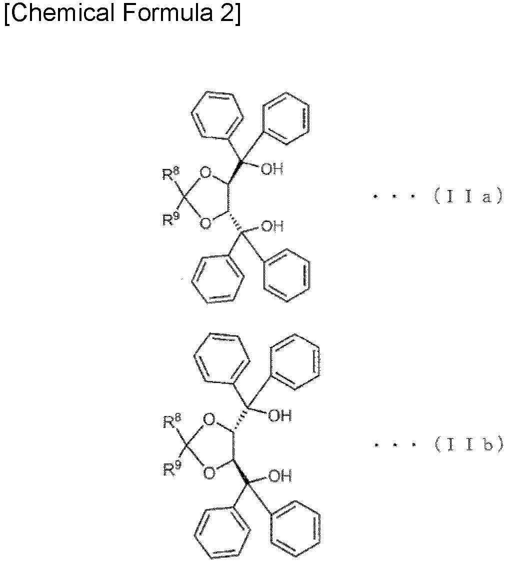 where in Formulae (IIa) and (IIb),
   R⁸ and R⁹ are a C₁-C₆-alkyl group, and R⁸ and R⁹ may form a ring together with a carbon atom to which they are bonded.

### ADVANTAGEOUS EFFECTS OF INVENTION

According to one aspect of the present invention, an (R)-enantiomer of a triazole derivative can be efficiently produced.

### DESCRIPTION OF EMBODIMENTS

A method for producing an (R)-enantiomer of a triazole derivative according to an embodiment of the present invention is described below in detail. Moreover, the embodiment explained below illustrates a representative example of the present invention, and it should not be interpreted that the scope of the present invention is narrowed by this embodiment.

A method for producing an (R)-enantiomer of a triazole derivative according to an embodiment of the present invention includes adding a chiral molecule to a triazole derivative in a solvent to perform co-crystallization, and separating a precipitated crystal and a residual liquid. Note that the term "production of an enantiomer" as used herein means that a racemate or a mixture of enantiomers is used as a starting material to achieve a state in which a desired enantiomer is contained in a larger amount. Thus, the term "production of an enantiomer" as used herein may also be expressed as separation of one enantiomer from a mixture of enantiomers. Note that all expressions do not necessarily mean that the obtained enantiomer is completely free of the other enantiomer. When the desired enantiomer is contained in a larger amount, an agricultural or horticultural chemical having higher activity than that obtained by using a racemate can be prepared.

Here, the enantiomer of the triazole derivative according to the present embodiment is an (-)-enantiomer (hereinafter referred to as a triazole derivative (-)-enantiomer) or an (+)-enantiomer (hereinafter referred to as a triazole derivative (+)-enantiomer) of a triazole derivative (hereinafter referred to as a triazole derivative (I)) represented by the following General Formula (I). The asterisk (*) in the following General Formula (I) refers to a chiral carbon atom. In the present specification, "(-)-enantiomer" refers to an enantiomer that rotates the oscillation plane of the linearly polarized light of sodium D lines to the left, and "(+)-enantiomer" refers to an enantiomer that rotates the oscillation plane of the linearly polarized light of sodium D lines to the right. As clarified in Examples described below, the triazole derivative (-)-enantiomer is an (R)-enantiomer of the triazole derivative (I), and the triazole derivative (+)-enantiomer is an (S)-enantiomer of the triazole derivative (I). In the present specification, the term "triazole derivative represented by General Formula (I)" or "triazole derivative (I)", when simply used, means the state in which the triazole derivative (-)-enantiomer and the triazole derivative (+)-enantiomer are not separated. Accordingly, the "triazole derivative represented by General Formula (I)" and the "triazole derivative (I)" in the present specification mean a mixture of the triazole derivative (-)-enantiomer and the triazole derivative (+)-enantiomer, and typically mean a racemate of the triazole derivative (I). 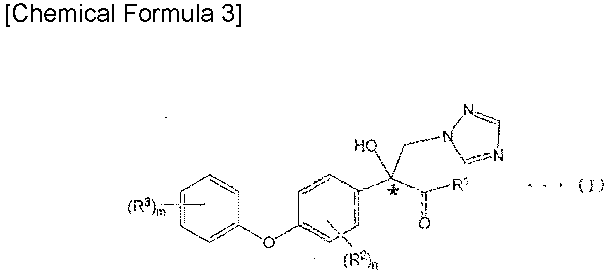 In General Formula (I), R¹ is -OR⁴ or -NR⁵R⁶, preferably -OR⁴.

R⁴, R⁵, and R⁶ are each independently hydrogen, a C₁-C₆-alkyl group, a C₂-C₆-alkenyl group, a C₂-C₆-alkynyl group, a C₃-C₈-cycloalkyl group, a C₃-C₈-cycloalkyl-C₁-C₄-alkyl group, a phenyl group, a phenyl-C₁-C₄-alkyl group, a phenyl-C₂-C₄-alkenyl group, or a phenyl-C₂-C₄-alkynyl group. R⁵ and R⁶ may form a ring together with the nitrogen atoms to which R⁵ and R⁶ are bonded.

The C₁-C₆-alkyl group is a linear or branched alkyl group having 1 to 6 carbon atoms, and includes, for example, a methyl group, an ethyl group, a 1-methylethyl group, a 1,1-dimethylethyl group, a propyl group, a 1-methylpropyl group, a 2-methylpropyl group, a 1,1-dimethylpropyl group, a 2,2-dimethylpropyl group, a 1-ethylpropyl group, a butyl group, a 1-methylbutyl group, a 2-methylbutyl group, a 3-methylbutyl group, a 3,3-dimethylbutyl group, a 2,2-dimethylbutyl group, a 1,1-dimethylbutyl group, a 1-ethylbutyl group, a 2-ethylbutyl group, a pentyl group, a 1-methylpentyl group, a 2-methylpentyl group, a 3-methylpentyl group, and a 4-methylpentyl group.

The C₂-C₆-alkenyl group is a linear or branched alkenyl group having 2 to 6 carbon atoms, and includes, for example, an ethenyl group, a 2 - propenyl group, a 1-methyl-2-propenyl group, a 2-methyl-2-propenyl group, a 1-butenyl group, a 2-butenyl group, a 3-methyl-2-butenyl group, a 1-methyl-2-butenyl group, a 3-butenyl group, a 1-pentenyl group, a 2-pentenyl group, a 1-hexenyl group, and a 5-hexenyl group.

The C₂-C₆-alkynyl group is a linear or branched alkynyl group having 2 to 6 carbon atoms, and includes, for example, an ethynyl group, a 1-propynyl group, a 2-propynyl group, a 1-butynyl group, a 2-butynyl group, a 3-butynyl group, a pentynyl group, and a 1-hexynyl group.

The C₃-C₈-cycloalkyl group is a cyclic alkyl having 3 to 8 carbon atoms, and includes, for example, a cyclopropyl group, a cyclobutyl group, a cyclopentyl group, a cyclohexyl group, a cycloheptyl group, and a cyclooctyl group.

The C₃-C₈-cycloalkyl-C₁-C₄-alkyl group indicates that a cyclic cycloalkyl group having 3 to 8 carbon atoms is bonded to a linear or branched alkyl group having 1 to 4 carbon atoms. Examples thereof include a cyclopropylmethyl group, a cyclobutylmethyl group, a cyclopentylmethyl group, a cyclohexylmethyl group, a 2-cyclopropylethyl group, a 1-cyclopropylethyl group, a 2-cyclohexylethyl group, a 3-cyclopropylpropyl group, a 2-cyclopropylpropyl group, and a 4-cyclopropylbutyl group.

The phenyl-C₁-C₄-alkyl group is a linear or branched alkyl group having 1 to 4 carbon atoms which is substituted with a phenyl group, and includes, for example, a phenylmethyl group, a 2 -phenylethyl group, a 3-phenylpropyl group, and a 4-phenylbutyl group.

The phenyl-C₂-C₄ -alkenyl group consists of a phenyl group bonded to a linear or branched alkenyl group having 2 to 4 carbon atoms, and includes, for example, a phenylethenyl group, a phenyl-1-propenyl group, a phenylisopropenyl group, and a phenylbutenyl group.

The phenyl-C₂-C₄-alkynyl group is a phenyl group bonded to an alkynyl group having 2 to 4 carbon atoms, and includes, for example, a phenylethynyl group, a phenyl-1-propynyl group, a phenyl-2-propynyl group, a phenyl-1-butynyl group, a phenyl-2-butynyl group, and a phenyl-3-butynyl group.

R⁴ is preferably a C₁-C₆-alkyl group.

The aliphatic groups in R¹, R⁴, R⁵, and R⁶ may have 1, 2, 3 or a possible maximum number of the same or different groups R^{a}s, and R^{a}s are each independently selected from a halogen group, a cyano group, a nitro group, a C₁-C₄-alkoxy group, and a C₁-C₄-haloalkoxy group.

Examples of the halogen group include a chlorine group, a bromine group, an iodine group, and a fluorine group. Examples thereof include a chloromethyl group, a 2-chloroethyl group, a 2,3-dichloropropyl group, a bromomethyl group, a chlorodifluoromethyl group, a trifluoromethyl group, and a 3,3,3-trifluoropropyl group.

The C₁-C₄-alkoxy group is a linear or branched alkoxy group having 1 to 4 carbon atoms, and includes, for example, a methoxy group, an ethoxy group, an n-propoxy group, an isopropoxy group, an n-butoxy group, a sec-butoxy, and a tert-butoxy group.

The C₁-C₄-haloalkoxy group is the above-described C₁-C₄-alkoxy group substituted with one or more halogen atoms at substitutable positions, and if there are two or more halogen substituents, the halogen groups may be the same or different.

R² is a halogen group, a cyano group, a nitro group, phenyl group, a phenyl-oxy group, a C₁-C₄-alkyl group, a C₁-C₄-haloalkyl group, a C₁-C₄-alkoxy group, a C₁-C₄-haloalkoxy group, -SOR⁷, or -SFs.

The halogen group, C₁-C₄-alkyl group, C₁-C₄-haloalkyl group, C₁-C₄-alkoxy group, and C₁-C₄-haloalkoxy group are the groups listed as examples of the organic groups represented by R^{a}.

R² is preferably a halogen group, a cyano group, a C₁-C₄-alkyl group, a C₁-C₄-haloalkyl group, a C₁-C₄-alkoxy group, -SOR⁷, or -SFs, and more preferably a halogen group, a cyano group, a C₁-C₄-alkyl group, a C₁-C₄-haloalkyl group, or a C₁-C₄-alkoxy group.

R⁷ is a C₁-C₄-alkyl group or a C₁-C₄-haloalkyl group. The substitution position of R² is the 2nd, 3rd, 5th, or 6th position, preferably the 2nd position. The n is 0, 1, 2, 3, or 4, preferably 1.

R³ is a halogen group, a cyano group, a nitro group, an amino group, a phenyl group, a phenyl-oxy group, a C₁-C₄-alkyl group, a C₁-C₄-haloalkyl group, a C₁-C₄-alkoxy group, a C₁-C₄-haloalkoxy group, a C₁-C₄-alkylamino group, a C₁-C₄-dialkylamino group, a C₁-C₄-alkylacylamino group, -SOR⁷, or - SFs, and the halogen group, C₁-C₄-alkyl group, C₁-C₄-haloalkyl group, C₁-C₄-alkoxy group, C₁-C₄-haloalkoxy group, and -SOR⁷ are the groups listed as the examples of the organic group represented by R².

R³ is preferably a halogen group, a nitro group, an amino group, a C₁-C₄-alkyl group, a C₁-C₄-haloalkyl group, a C₁-C₄-alkoxy group, a C₁-C₄-haloalkoxy group, a C₁-C₄-alkylamino group, a C₁-C₄-dialkylamino group, a C₁-C₄-alkylacylamino group, -SOR⁷, or -SFs, and more preferably a halogen group, a C₁-C₄-alkyl group, a C₁-C₄-haloalkyl group, a C₁-C₄-alkoxy group, or a C₁-C₄-haloalkoxy group.

The C₁-C₄-alkylamino group is an amino group in which one of the hydrogen atoms of the amino group is replaced by a linear or branched alkyl group having 1 to 4 carbon atoms, and includes, for example, a methylamino group, an ethylamino group, a n-propylamino group, an isopropylamino group, and a tert-butylamino group.

The C₁-C₄-dialkylamino group is an amino group in which both of the two hydrogen atoms of the amino group are replaced by linear or branched alkyl groups having 1 to 4 carbon atoms, and includes, for example, an N,N-dimethylamino group, an N,N-diethylamino group, an N,N-di-n-propylamino group, an N,N-di-isopropylamino group, and an N,N-di-tert-butylamino group.

The C₁-C₄-alkylacylamino group is an amino group in which one or two of the hydrogen atoms of the amino group are replaced by a linear or branched alkylacyl group having 1 to 4 carbon atoms, and includes, for example, a methylacylamino group, an ethylacylamino group, a n-propylacylamino group, an isopropylacylamino group, a tert-butylacylamino group, an N,N-dimethylacylamino group, an N,N-diethylacylamino group, an N,N-di-n-propylacylamino group, an N,N-diisopropylacylamino group, and an N,N-di-tert-butylacylamino group.

The cycloalkyl group or phenyl group moieties in R⁴, R⁵, and R⁶, or the phenyl group moiety in R³ may have 1, 2, 3, 4, 5 or a possible maximum number of the same or different groups R^{b}s, and R^{b}s are each independently selected from a halogen group, a cyano group, a nitro group, a C₁-C₄-alkyl group, a C₁-C₄-alkoxy group, a C₁-C₄-haloalkyl group, and a C₁-C₄-haloalkoxy group. The halogen group, C₁-C₄-alkyl group, C₁-C₄-alkoxy group, C₁-C₄-haloalkyl group, and C₁-C₄-haloalkoxy group are the groups listed as examples of the organic groups represented by R^{a}.

From the above, a preferred embodiment of the triazole derivative is the triazole derivative represented by General Formula (I), in which R¹ is -OR⁴; R² is a halogen group, a cyano group, a C₁-C₄-alkyl group, a C₁-C₄-haloalkyl group, a C₁-C₄-alkoxy group, -SOR⁷, or -SFs; and R³ is a halogen group, a nitro group, a cyano group, an amino group, a C₁-C₄-alkyl group, a C₁-C₄-haloalkyl group, a C₁-C₄-alkoxy group, a C₁-C₄-haloalkoxy group, a C₁-C₄-alkylamino group, a C₁-C₄-dialkylamino group, a C₁-C₄-alkylacylamino group, -SOR⁷, or -SFs.

A preferred aspect of the triazole derivative is the triazole derivative represented by General Formula (I), in which R¹ is -OR⁴, R⁴ is a C₁-C₆-alkyl group; R² is a halogen group, a cyano group, a C₁-C₄-alkyl group, a C₁-C₄-haloalkyl group, or a C₁-C₄-alkoxy group; R³ is a halogen group, a cyano group, a C₁-C₄-alkyl group, a C₁-C₄-haloalkyl group, a C₁-C₄-alkoxy group, or a C₁-C₄-haloalkoxy group.

### 1. Co-crystallization of triazole derivative and chiral molecule

As described above, the triazole derivative (I) is typically a racemate of the triazole derivative (I). The racemate of the triazole derivative (I) can be prepared according to the method described in Patent Document 1, for example. Note that in a case where the triazole derivative (I) is produced according to the method described in Patent Document 1, the resulting triazole derivative (I) is a racemate. The prepared racemate of the triazole derivative (I) is mixed with a chiral molecule represented by the following General Formula (IIa) or (IIb) in a solvent to be crystallized, whereby a co-crystal of one enantiomer of the triazole derivative (I) and the chiral molecule can be produced.

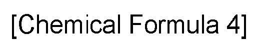

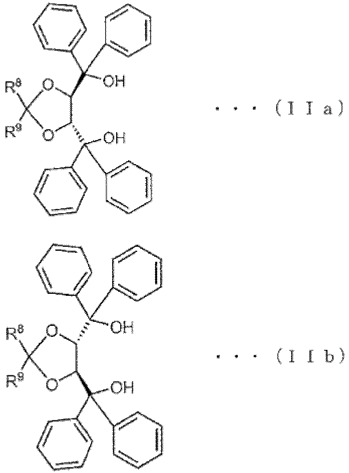

where in General Formulae (IIa) and (IIb), R⁸ and R⁹ each are a C₁-C₆-alkyl group, and R⁸ and R⁹ may form a ring together with a carbon atom to which they are bonded.

The C₁-C₆-alkyl group is a linear or branched alkyl group having 1 to 6 carbon atoms, and examples thereof include a methyl group, an ethyl group, a 1-methylethyl group, a 1,1-dimethylethyl group, a propyl group, a 1-methylpropyl group, a 2-methylpropyl group, a 1,1-dimethylpropyl group, a 2,2-dimethylpropyl group, a 1-ethylpropyl group, a butyl group, a 1-methylbutyl group, a 2-methylbutyl group, a 3-methylbutyl group, a 3,3-dimethylbutyl group, a 2,2-dimethylbutyl group, a 1,1-dimethylbutyl group, a 1-ethylbutyl group, a 2-ethylbutyl group, a pentyl group, a 1-methylpentyl group, a 2-methylpentyl group, a 3-methylpentyl group, and a 4-methylpentyl group.

The chiral molecule represented by General Formula (IIa) is preferably a chiral molecule represented by the following General Formula (IIIa) or (IVa). 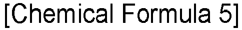 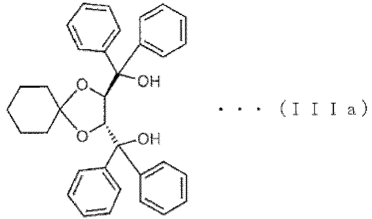 (General Formula (IIIa): ((2R,3R)-1,4-dioxaspiro[4.5]decane-2,3-diyl)bis(diphenylmethanol)) 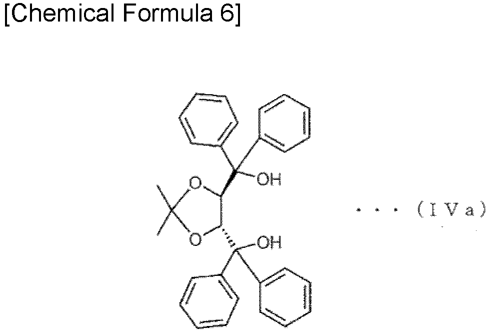 (General Formula (IVa): ((4R,5R)-2,2-dimethyl-1,3-dioxolane-4,5-diyl)bis(diphenylmethanol))

The chiral molecule represented by General Formula (IIb) is preferably a chiral molecule represented by the following General Formula (IIIb) or (IVb). 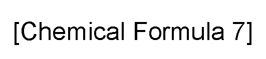 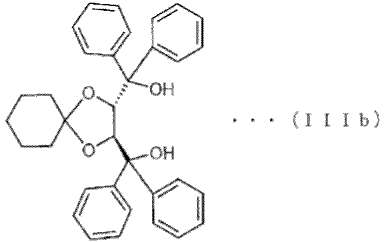 (General Formula (IIIb): ((2S,3S)-1,4-dioxaspiro[4.5]decane-2,3-diyl)bis(diphenylmethanol)) 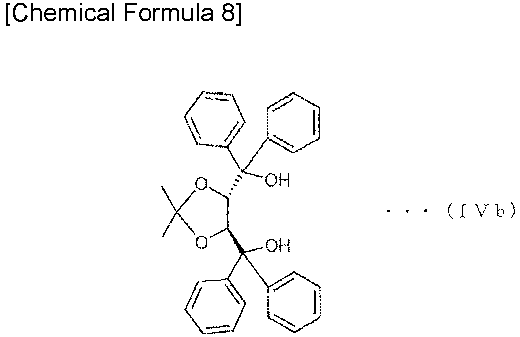 (General Formula (IVb): ((4R,5R)-2,2-dimethyl-1,3-dioxolane-4,5-diyl)bis(diphenylmethanol))

The amount of the chiral molecule added is preferably from 0.01 to 100 mol, more preferably from 0.1 to 10 mol, and still more preferably from 0.5 to 5 mol, per 1 mol of the triazole derivative (I).

The solvent used is not particularly limited, and preferred examples thereof include alcohols such as methanol and ethanol. Other examples thereof include aromatic hydrocarbons such as benzene, toluene, xylene, and chlorobenzene; amides such as N,N-dimethylacetamide, N-methylpyrrolidone, and N,N-dimethylformaldehyde; and dimethylsulfoxide. The solubility range of the triazole derivative (I) may vary depending on the type of the solvent, and thus the amount of the solvent used is appropriately determined depending on the solubility range of the triazole derivative (I). For example, in a case where methanol is used as the solvent, the amount of methanol is preferably from 10 to 1000000 mol, more preferably from 50 to 500000 mol, and still more preferably from 100 to 10000 mol, per 1 mol of the triazole derivative (I).

The solvent may be heated or need not be heated. In a case where the solvent is not heated, after the triazole derivative enantiomer is dissolved, the solution is allowed to stand for a while to precipitate a crystal. When crystallization is performed without heating, a crystal having a higher abundance ratio of one enantiomer can be produced. In a case where the solvent is heated, the solvent may be heated in advance before addition of the triazole derivative (I), or may be heated after addition of the triazole derivative (I). In a case where heating is performed after addition of the triazole derivative (I), heating may be performed after addition of both the triazole derivative (I) and the chiral molecule. Then, the heated solvent to which the chiral molecule has been added is cooled to perform crystallization. The temperature of the solvent after heating is not particularly limited, and may be, for example, from 25 to 64°C, preferably from 40 to 64°C, and more preferably from 45 to 60°C. The temperature of the solvent after cooling is not particularly limited, and is preferably from -50 to 25°C, and more preferably from 0 to 25°C. Typically, it may be room temperature (for example, 25°C). In a case where the temperature is set to room temperature or higher, an excessive cooling operation is not necessary, and the solvent may be allowed to stand at room temperature, for example.

The method of crystallization after mixing the triazole derivative (I) and the chiral molecule, that is, the method of precipitating a co-crystal is not limited thereto, and a precipitation method known in the related art such as a preferential crystallization method, a diastereomer method, an asymmetric crystallization method, or a method of evaporating a solvent can be adopted. For example, in a method of co-crystal precipitation by evaporating a solvent, one enantiomer in the obtained crystal has a larger ratio.

Although both the triazole derivative enantiomers are amorphous compounds, addition of the chiral molecule described above to the triazole derivative (I) allows crystallization of a co-crystal of one enantiomer of the triazole derivative (I) and a chiral molecule. As a result, when the obtained crystal is separated from the residual liquid, a state in which one enantiomer is contained in a larger amount can be achieved. On the other hand, the residual liquid obtained by separating the crystal contains a larger amount of the other enantiomer of the triazole derivative (I), and thus a state in which the other enantiomer is contained in a larger amount can be achieved by fractionation of the residual liquid after the crystal formation.

Further, the enantiomer of the triazole derivative (I) forming the co-crystal can be changed by selection of the chiral molecule. For example, in a case where the chiral molecule represented by General Formula (IIa) is used as the chiral molecule, a co-crystal with the (R)-enantiomer of the triazole derivative (I) is formed. On the other hand, in a case where the chiral molecule represented by General Formula (IIb), which is an enantiomer of the chiral molecule of General Formula (IIa), is used, a co-crystal with the (S)-enantiomer of the triazole derivative (I) is formed.

The co-crystal of the triazole derivative (I) and the chiral molecule, as obtained above, and the residual liquid are separated, for example, by filtration.

### 2. Generation of (R)-enantiomer of triazole derivative 1

As described above, depending on the chiral molecule used, which of the (R)-enantiomer or the (S)-enantiomer of the triazole derivative (I) is co-crystallized with the chiral molecule varies. In the present section, the case of using the chiral molecule represented by General Formula (IIa) with which the (R)-enantiomer of the triazole derivative (I) is co-crystallized will be described. In the case of using the chiral molecule, separating the precipitated crystal and the residual liquid can be described as separating the precipitated crystal and the residual liquid to obtain the crystal. Note that depending on the chiral molecule, the chiral molecule represented by General Formula (IIa) can also be co-crystallized with the (S)-enantiomer of the triazole derivative (I). However, in a case where co-crystallization is performed for the racemate, the (R)-enantiomer is preferentially co-crystallized.

The separated crystal may contain not only the (R)-enantiomer of the triazole derivative (I) but also the (S)-enantiomer of the triazole derivative, but the content of the (R)-enantiomer is higher than that of the (S)-enantiomer. Typically, the ratio of the content of the (R)-enantiomer to the total amount of the content of the (R)-enantiomer and the content of the (S)-enantiomer may be more than 50%, 70% or more, 95% or more, or 100%.

The ratio of the (R)-enantiomer to the (S)-enantiomer contained in the separated crystal can be confirmed by a method in which the obtained crystal is dissolved in a solvent and each enantiomer is preparatively separated by chiral chromatography. The preparative separation by chiral chromatography is performed with reference to the method described in Patent Document 2, for example.

To increase the content ratio of the target (R)-enantiomer of the triazole derivative (I), a step of removing the chiral molecule in the crystal from the obtained co-crystal may be added. Examples of the method for removing the chiral molecule include silica gel chromatography. Consequently, the (R)-enantiomer of the triazole derivative (I) from which the chiral molecule has been removed can be generated.

### 3. Generation of (R)-enantiomer of triazole derivative 2

As described above, depending on the chiral molecule used, which of the (R)-enantiomer or the (S)-enantiomer of the triazole derivative (I) is co-crystallized with the chiral molecule varies. In the present section, the case of using the chiral molecule represented by General Formula (IIb) with which the (S)-enantiomer of the triazole derivative (I) is co-crystallized will be described. In a case where the chiral molecule is used, separating the precipitated crystal and the residual liquid can be described as separating the precipitated crystal and the residual liquid and fractionating the filtrate after crystallization.

In the case of using the chiral molecule represented by General Formula (IIb), a larger amount of the (S)-enantiomer forms a crystal as compared with the (R)-enantiomer. When the obtained crystal is removed by filtration of the crystal, the filtrate after the filtration of the crystal (i.e., the residual liquid after the crystal formation) eventually contains a larger amount of the (R)-enantiomer. Thus, when an amorphous solid is generated from the filtrate, a solid containing a larger amount of the (R)-enantiomer of the triazole derivative can be produced. Examples of the method for generating an amorphous solid include evaporation of the solvent and column chromatography.

Although the obtained amorphous solid may contain not only the (R)-enantiomer of the triazole derivative (I) but also the (S)-enantiomer of the triazole derivative, the content of the (R)-enantiomer is higher than that of the (S)-enantiomer. Typically, the ratio of the content of the (R)-enantiomer to the total amount of the content of the (R)-enantiomer and the content of the (S)-enantiomer may be more than 50%, 70% or more, 95% or more, or 100%.

In addition, the filtrate after filtration of the obtained amorphous solid or co-crystal may also contain the chiral molecule used for co-crystallization. The chiral molecule is separated by silica gel chromatography or the like, whereby the triazole derivative (I) not containing the chiral molecule can be produced.

### Summary

A method for producing an (R)-enantiomer of a triazole derivative according to a first embodiment of the present invention includes adding a chiral molecule represented by the above-described General Formula (IIa) or (IIb) to a triazole derivative represented by the above-described General Formula (I) in a solvent to perform crystallization, and separating a precipitated crystal and a residual liquid.

In a method for producing an (R)-enantiomer of a triazole derivative according to a second embodiment of the present invention, in addition to the configuration of the first embodiment of the present invention, the chiral molecule is a chiral molecule represented by General Formula (IIa), and the (R)-enantiomer of the triazole derivative is generated from the separated crystal.

In a method for producing an (R)-enantiomer of a triazole derivative according to a third embodiment of the present invention, in addition to the configuration of the second embodiment of the present invention, the chiral molecule in the separated crystal is removed by silica gel chromatography to generate the (R)-enantiomer of the triazole derivative.

In a method for producing an (R)-enantiomer of a triazole derivative according to a fourth embodiment of the present invention, in addition to the configuration of the second embodiment of the present invention, the chiral molecule represented by General Formula (IIa) is ((2R,3R)-1,4-dioxaspiro[4.5]decane-2,3-diyl)bis(diphenylmethanol) or ((4R,5R)-2,2-dimethyl-1,3-dioxolane-4,5-diyl)bis(diphenylmethanol).

In a method for producing an (R)-enantiomer of a triazole derivative according to a fifth embodiment of the present invention, in addition to the configuration of the first embodiment of the present invention, the chiral molecule is a chiral molecule represented by General Formula (IIb), and the (R)-enantiomer of the triazole derivative is generated from the separated residual liquid.

In a method for producing an (R)-enantiomer of a triazole derivative according to a sixth embodiment of the present invention, in addition to the configuration of the fifth embodiment of the present invention, an amorphous solid is generated from the residual liquid, and the amorphous solid is separated as the (R)-enantiomer of the triazole derivative.

In a method for producing an (R)-enantiomer of a triazole derivative according to a seventh embodiment of the present invention, in addition to the configuration of the fifth embodiment of the present invention, the chiral molecule represented by General Formula (IIb) is ((2S,3S)-1,4-dioxaspiro[4.5]decane-2,3-diyl)bis(diphenylmethanol) or ((4S,5S)-2,2-dimethyl-1,3-dioxolane-4,5-diyl)bis(diphenylmethanol).

In a method for producing an (R)-enantiomer of a triazole derivative according to an eighth embodiment of the present invention, in addition to the configuration of the first embodiment of the present invention, the method includes heating a solvent before or after adding a chiral molecule, in which the crystallization is performed by cooling the heated solvent with the chiral molecule added.

In a method for producing an (R)-enantiomer of a triazole derivative according to a ninth embodiment of the present invention, in addition to the configuration of any one of the first to eighth embodiments of the present invention, in General Formula (I),
R¹ is -OR⁴;
R² is a halogen group, a cyano group, a C₁-C₄-alkyl group, a C₁-C₄-haloalkyl group, a C₁-C₄-alkoxy group, -SOR⁷, or -SFs; and
R³ is a halogen group, a nitro group, a cyano group, an amino group, a C₁-C₄-alkyl group, a C₁-C₄-haloalkyl group, a C₁-C₄-alkoxy group, a C₁-C₄-haloalkoxy group, a C₁-C₄-alkylamino group, a C₁-C₄-dialkylamino group, a C₁-C₄-alkylacylamino group, -SOR⁷, or -SFs.

In a method for producing an (R)-enantiomer of a triazole derivative according to a tenth embodiment of the present invention, in addition to the configuration of the ninth embodiment of the present invention, in General Formula (I),
R⁴ is a C₁-C₆-alkyl group;
R² is a halogen group, a cyano group, a C₁-C₄-alkyl group, a C₁-C₄-haloalkyl group, or a C₁-C₄-alkoxy group; and
R³ is a halogen group, a cyano group, a C₁-C₄-alkyl group, a C₁-C₄-haloalkyl group, a C₁-C₄-alkoxy group, or a C₁-C₄-haloalkoxy group.

Embodiments of the present invention will be further described in detail using the examples below. Needless to say, the present invention is not limited to the following examples, and details of the present invention can be in various manners. Furthermore, the present invention is not limited to the embodiments described above, and it may be varied in various ways within the scope of the claims. Thus, an embodiment achieved by appropriately combining technical means described herein will be included in the technical scope of the present invention. In addition, the contents of all the literatures referred herein are incorporated by reference in their entirety.

### EXAMPLES

### Reference Example 1: Investigation of chiral molecule

To search for a compound that preferentially forms a co-crystal with an (-)-enantiomer of methyl 2-(2-chloro-4-(4-chlorophenoxyphenyl)phenyl)-2-hydroxy-3-(1H-1,2,4-triazol-1-yl) propanoate (hereinafter referred to as compound 1), the following chiral molecules 1 to 13 were used to attempt co-crystallization with the (-)-enantiomer of compound 1 and co-crystallization with the (+)-enantiomer of compound 1.

Compound 1 was synthesized with reference to the method described in Patent Document 1. In addition, each enantiomer was preparatively separated by chiral chromatography from the racemate of compound 1 according to the method described in Patent Document 2. One enantiomer was dissolved in methanol according to the formulation listed in Table 1 or 2 below. One of the chiral molecules 1 to 13 was added thereto and dissolved according to the formulation listed in Table 1 or 2, and the mixture was allowed to stand at 25°C to attempt to precipitate a crystal. The results are listed in Tables 1 and 2. The mixture that became oil was determined to be incapable of co-crystal formation ("NO" in the tables). In a case where a crystal or a solid was precipitated, the crystal or solid was subjected to a powder X-ray diffraction (PXRD) measurement. When a peak different from that derived from the chiral molecule was detected, it was determined that co-crystal formation was possible ("YES" in the tables), and when a peak derived from the chiral molecule was detected, it was determined that no co-crystal was formed and thus co-crystal formation was not possible ("NO" in the tables).

As listed in Table 1, it was found that only chiral molecules 7 and 10 were capable of co-crystallization with the (-)-enantiomer. In addition, as listed in Table 2, it was found that only chiral molecules 7 and 10 were capable of co-crystallization with the (+)-enantiomer.
· Chiral molecule 1: (R)-(+)-1,1-bi-2-naphthol 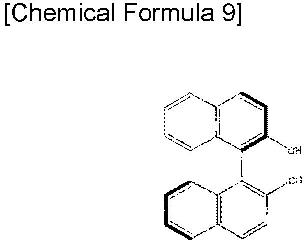
· Chiral molecule 2: (R)-3,3-dibromo-1,1-bi-2-naphthol 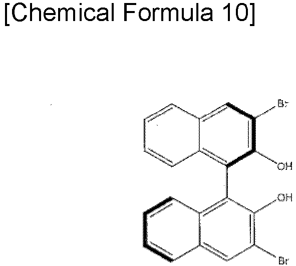
· Chiral molecule 3: (R)-2,2,3,3-tetrahydro-1,1-spirobi[indene]-7,7-diol 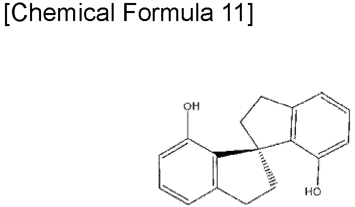
· Chiral molecule 4: (R)-(-)-1-phenylethane-1,2-diol 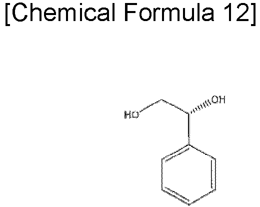
· Chiral molecule 5: (R)-1-[3,5-bis(trifluoromethyl)phenyl]ethanol 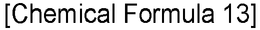 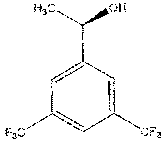
· Chiral molecule 6: (3S,4S)-(phenylmethyl)-3,4-pyrrolidinediol 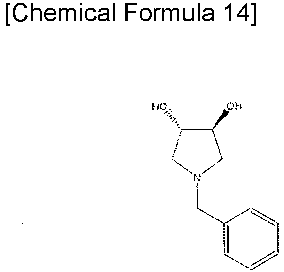
· Chiral molecule 7: ((4R,5R)-2,2-dimethyl-1,3-dioxolane-4,5-diyl)bis(diphenylmethanol) 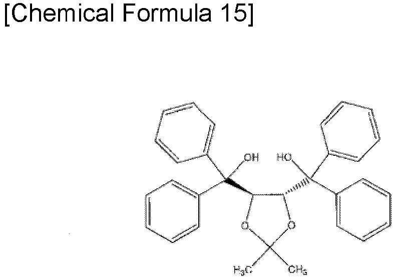
· Chiral molecule 8: (1R,2R)- N,N-bis(3,5-di-felt-butylsalicylidene) 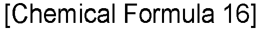 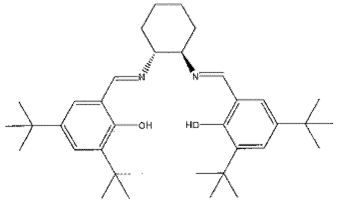
· Chiral molecule 9: (11bR)-4-hydroxy-4-oxido-dinaphtho[2,1-d:1,2-f][1,3,2]dioxaphosphepin 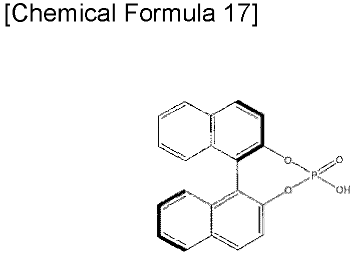
· Chiral molecule 10: ((2R,3R)-1,4-dioxaspiro[4.5]decane-2,3-diyl)bis(diphenylmethanol) 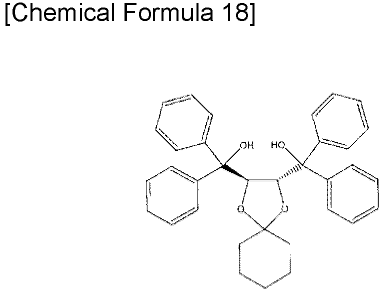
· Chiral molecule 11: (1R,2R)-1,2-diphenylethylenediamine 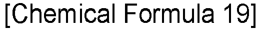 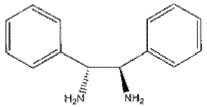
· Chiral molecule 12: 4-fluoro-L-phenylalanine 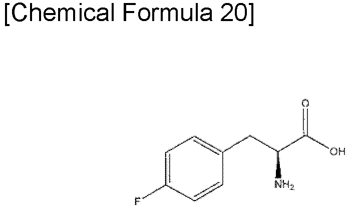
· Chiral molecule 13: (R)-(+)-1,1'-binaphthyl-2,2'-diamine

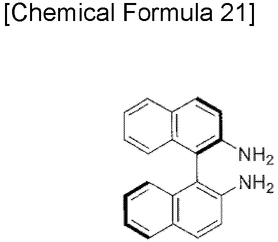

**[Table 1]**

| Co-crystallization with (-)-enantiomer | | | | |
|---|---|---|---|---|
| Chiral molecule | Addition amount of (-)-enantiomer (mg) | Addition amount of chiral molecule (mg) | Addition amount of methanol (mg) | Result |
| 1 | 20.3 | 14.1 | 3 | No |
| 2 | 19.9 | 22.0 | 3 | No |
| 3 | 20.0 | 12.5 | 3 | No |
| 4 | 20.2 | 6.8 | 3 | No |
| 5 | 20.1 | 12.7 | 3 | No |
| 6 | 20.4 | 9.5 | 3 | No |
| 7 | 19.9 | 23 | 3 | Yes |
| 8 | 19.9 | 26.8 | 4 | No |
| 9 | 20.0 | 17.1 | 3 | No |
| 10 | 20.2 | 24.7 | 3 | Yes |
| 11 | 20.3 | 10.7 | 3 | No |
| 12 | 20.3 | 8.7 | 3 | No |
| 13 | 20.3 | 14.0 | 3 | No |

**[Table 2]**

| Co-crystallization with (+)-enantiomer | | | | |
|---|---|---|---|---|
| Chiral molecule | Addition amount of (+)-enantiomer (mg) | Addition amount of chiral molecule (mg) | Addition amount of methanol (mg) | Result |
| 1 | 20.4 | 14.3 | 3 | No |
| 2 | 20.1 | 22.1 | 3 | No |
| 3 | 20.1 | 12.5 | 3 | No |
| 4 | 19.9 | 6.8 | 3 | No |
| 5 | 19.9 | 12.7 | 3 | No |
| 6 | 20.2 | 9.5 | 3 | No |
| 7 | 19.9 | 22.9 | 3 | Yes |
| 8 | 20.2 | 26.9 | 4 | No |
| 9 | 19.9 | 16.9 | 3 | No |
| 10 | 19.9 | 25.0 | 3 | Yes |
| 11 | 20.1 | 10.2 | 3 | No |
| 12 | 19.8 | 8.9 | 3 | No |
| 13 | 19.7 | 14.0 | 3 | No |

### Reference Example 2: Determination of structure of absolute configuration of (-)-enantiomer

The enantiomer of compound 1 is an amorphous compound and thus is not capable of crystallization, and it was impossible so far to know the structure of the absolute configuration by crystal structure analysis. As described in Reference Example 1, the use of a specific chiral molecule enabled the crystallization of the (-)-enantiomer as a co-crystal with the chiral molecule, and thus crystal structure analysis was performed. Specifically, 20 mg of the (-)-enantiomer of compound 1 was dissolved in 3 mL of methanol, 24.8 mg of the chiral molecule 10 was added thereto, and the mixture was allowed to stand at 25°C until a white crystal was precipitated. The precipitated white needle-like crystal was subjected to single crystal X-ray crystal structure analysis. The result of the structure analysis shows that the (-)-enantiomer of compound 1 was determined to be the (R)-enantiomer of compound 1. Thus, in the following examples, the (-)-enantiomer is described as the (R)-enantiomer and the (+)-enantiomer is described as the (S)-enantiomer.

### Example 1

100 mg of the racemate of compound 1 was dissolved in 3 mL of methanol at 60°C. 123 mg of the chiral molecule 10 was added thereto and dissolved, and the mixture was allowed to stand at 25°C to obtain a precipitated white solid. The obtained white solid was preparatively separated into the (R)-enantiomer and the (S)-enantiomer using CHIRALPAK IG, and the content ratio was analyzed based on the area ratio of the peaks. The analysis result showed that the content ratio of (R)-enantiomer : (S)-enantiomer was 21 : 8.

### (Analysis condition)

Column: CHIRALPAK IG (available from Daicel Corporation) 4.6 × 250 mm
Particle size: 5 µm
Mobile phase: MeOH
Flow rate: 1.0 mL/min
Column temperature: 40°C
Injection volume: 10 µL

### Example 2

50 mg of the racemate of compound 1 was dissolved in 1 mL of methanol at 25°C. 93 mg of the chiral molecule 10 was added thereto and dissolved, and the mixture was allowed to stand at 25°C to obtain a precipitated white solid. The obtained white solid was washed with cold methanol, and the washed white solid was preparatively separated into the (R)-enantiomer and the (S)-enantiomer in the same manner as in Example 1. The content ratio was analyzed based on the area ratio of the peaks. The analysis result showed that the proportion of the (R)-enantiomer was 99% or more.

### Example 3

50 mg of the racemate of compound 1 was dissolved in 0.5 mL of methanol at 60°C. 62 mg of ((2S,3S)-1,4-dioxaspiro [4.5]decane-2,3-diyl)bis(diphenylmethanol), which is an enantiomer of the chiral molecule 10, was added thereto and dissolved, and the mixture was allowed to stand at 25°C to obtain a precipitated white solid. The obtained white solid was washed with cold methanol and separated by filtration, and the obtained filtrate was preparatively separated into the (R)-enantiomer and the (S)-enantiomer in the same manner as in Example 1. The content ratio of the filtrate was analyzed based on the area ratio of the peaks. The analysis result showed that the content ratio of (R)-enantiomer : (S)-enantiomer was 4.6 : 1.

### INDUSTRIAL APPLICABILITY

The method for producing an (R)-enantiomer of a triazole derivative according to an embodiment of the present invention can be used for antimicrobial agents for agriculture or horticulture and protective agents for industrial materials containing the enantiomer as an active ingredient.

## Claims

1. A method for producing an (R)-enantiomer of a triazole derivative, the method comprising:
adding a chiral molecule represented by the following General Formula (IIa) or (IIb) to a triazole derivative represented by the following General Formula (I) in a solvent to perform crystallization; and
separating a precipitated crystal and a residual liquid: 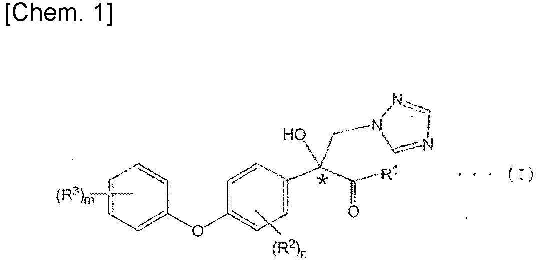 where in Formula (I),
R¹ is -OR⁴ or -NR⁵R⁶;
R⁴, R⁵, and R⁶ are each independently hydrogen, a C₁-C₆-alkyl group, a C₂-C₆-alkenyl group, a C₂-C₆-alkynyl group, a C₃-C₈-cycloalkyl group, a C₃-C₈-cycloalkyl-C₁-C₄-alkyl group, a phenyl group, a phenyl-C₁-C₄-alkyl group, a phenyl-C₂-C₄-alkenyl group, or a phenyl-C₂-C₄-alkynyl group, and R⁵ and R⁶ may form a ring together with a nitrogen atom to which they are bonded;
the aliphatic groups in R⁴, R⁵, and R⁶ may have 1, 2, 3 or a possible maximum number of the same or different groups R^{a}s, where R^{a}s are each independently selected from a halogen group, a cyano group, a nitro group, a C₁-C₄-alkoxy group, and a C₁-C₄-haloalkoxy group;
R² is a halogen group, a cyano group, a nitro group, a phenyl group, a phenyl-oxy group, a C₁-C₄-alkyl group, a C₁-C₄-haloalkyl group, a C₁-C₄-alkoxy group, a C₁-C₄-haloalkoxy group, -SOR⁷, or -SFs;
R³ is a halogen group, a cyano group, a nitro group, an amino group, a phenyl group, a phenyl-oxy group, a C₁-C₄-alkyl group, a C₁-C₄-haloalkyl group, a C₁-C₄-alkoxy group, a C₁-C₄-haloalkoxy group, a C₁-C₄-alkylamino group, a C₁-C₄-dialkylamino group, a C₁-C₄-alkylacylamino group, -SOR⁷, or -SFs;
the cycloalkyl group and phenyl group moieties of R⁴, R⁵, and R⁶ and the phenyl group moiety of R³ may have 1, 2, 3, 4, 5, or a possible maximum number of the same or different groups R^{b}s, where R^{b}s are each independently selected from a halogen group, a cyano group, a nitro group, a C₁-C₄-alkyl group, a C₁-C₄-alkoxy group, a C₁-C₄-haloalkyl group, and a C₁-C₄-haloalkoxy group;
R⁷ is a C₁-C₄-alkyl group or a C₁-C₄-haloalkyl group;
n is 0, 1, 2, 3, or 4;
m is 1, 2, 3, 4, or 5; and
the asterisk (*) refers to a chiral carbon atom, and 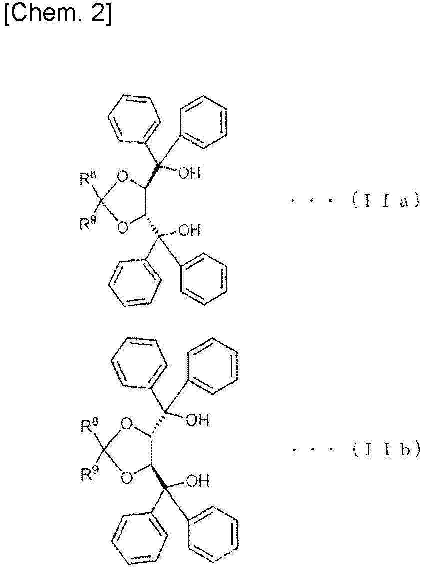 where in Formulae (IIa) and (IIb),
R⁸ and R⁹ are C₁-C₆-alkyl groups, and R⁸ and R⁹ may form a ring together with a carbon atom to which they are bonded.

2. The method for producing an (R)-enantiomer of a triazole derivative according to claim 1, wherein
the chiral molecule is a chiral molecule represented by General Formula (IIa), and
the (R)-enantiomer of the triazole derivative is generated from the separated crystal.

3. The method for producing an (R)-enantiomer of a triazole derivative according to claim 2, wherein
the chiral molecule in the separated crystal is removed by silica gel chromatography to generate the (R)-enantiomer of the triazole derivative.

4. The method for producing an (R)-enantiomer of a triazole derivative according to claim 2, wherein
the chiral molecule represented by General Formula (IIa) is ((2R,3R)-1,4-dioxaspiro[4.5]decane-2,3-diyl)bis(diphenylmethanol) or ((4R,5R)-2,2-dimethyl-1,3-dioxolane-4,5-diyl)bis(diphenylmethanol).

5. The method for producing an (R)-enantiomer of a triazole derivative according to claim 1, wherein
the chiral molecule is a chiral molecule represented by General Formula (IIb), and
the (R)-enantiomer of the triazole derivative is generated from the separated residual liquid.

6. The method for producing an (R)-enantiomer of a triazole derivative according to claim 5, wherein
an amorphous solid is generated from the residual liquid, and the amorphous solid is separated as the (R)-enantiomer of the triazole derivative.

7. The method for producing an (R)-enantiomer of a triazole derivative according to claim 5, wherein
the chiral molecule represented by General Formula (IIb) is ((2S,3S)-1,4-dioxaspiro[4.5]decane-2,3-diyl)bis(diphenylmethanol) or ((4S,5S)-2,2-dimethyl-1,3-dioxolane-4,5-diyl)bis(diphenylmethanol).

8. The method for producing an (R)-enantiomer of a triazole derivative according to claim 1, the method further comprising heating the solvent before or after addition of the chiral molecule, wherein the crystallization is performed by cooling the heated solvent with the chiral molecule added.

9. The method for producing an (R)-enantiomer of a triazole derivative according to any one of claims 1 to 8, wherein
in General Formula (I),
R¹ is -OR⁴;
R² is a halogen group, a cyano group, a C₁-C₄-alkyl group, a C₁-C₄-haloalkyl group, a C₁-C₄-alkoxy group, -SOR⁷, or -SFs; and
R³ is a halogen group, a nitro group, a cyano group, an amino group, a C₁-C₄-alkyl group, a C₁-C₄-haloalkyl group, a C₁-C₄-alkoxy group, a C₁-C₄-haloalkoxy group, a C₁-C₄-alkylamino group, a C₁-C₄-dialkylamino group, a C₁-C₄-alkylacylamino group, -SOR⁷, or -SFs.

10. The method for producing an (R)-enantiomer of a triazole derivative according to claim 9, wherein
in General Formula (I),
R⁴ is a C₁-C₆-alkyl group;
R² is a halogen group, a cyano group, a C₁-C₄-alkyl group, a C₁-C₄-haloalkyl group, or a C₁-C₄-alkoxy group; and
R³ is a halogen group, a cyano group, a C₁-C₄-alkyl group, a C₁-C₄-haloalkyl group, a C₁-C₄-alkoxy group, or a C₁-C₄-haloalkoxy group.
